Europäisches Patentamt

European Patent Office

Office européen des brevets

(11) Publication number: **0 029 976**
**B1**

(12) **EUROPEAN PATENT SPECIFICATION**

(45) Date of publication of patent specification: **23.01.85**

(21) Application number: **80107281.0**

(22) Date of filing: **21.11.80**

(51) Int. Cl.⁴: **C 12 N 1/20,** C 12 N 9/00, C 12 N 9/12, C 12 N 9/04 // C12R1/07

(54) **A biologically pure culture of strain IFO 14093 and a process for producing an intracellular component herefrom.**

(30) Priority: **22.11.79 JP 151660/79**
**18.04.80 JP 52198/80**
**18.04.80 JP 52199/80**
**25.04.80 JP 55647/80**
**12.05.80 JP 63180/80**
**21.05.80 JP 68018/80**
**10.07.80 JP 94868/80**

(43) Date of publication of application:
**10.06.81 Bulletin 81/23**

(45) Publication of the grant of the patent:
**23.01.85 Bulletin 85/04**

(84) Designated Contracting States:
**DE FR GB IT SE**

(56) References cited:

EXPERIENTIA, vol. 29, no. 8, 15th August 1973, pages 941-942, H. HENGARTNER et al.:
"Isolation of different thermophilic enzymes from Bacillus stearothermophilus"

(73) Proprietor: **UNITIKA LTD.**
**No. 50, Higashihonmachi 1-chome**
**Amagasaki-shi Hyogo (JP)**

(72) Inventor: **Nakajima, Hiroshi**
**No. 80-4, Enba Makishima-cho**
**Uji-Shi Kyoto (JP)**
Inventor: **Nagata, Kazuhiko**
**No. 15-12, Shironosato**
**Nagaokakyo-shi Kyoto (JP)**
Inventor: **Kageyama, Masao**
**No. 55, Uji-Kageyama**
**Uji-shi Kyoto (JP)**
Inventor: **Suga, Toyohiko**
**No. 21-4, Daigo-Uenoyama-cho**
**Fushimi-ku Kyoto-shi (JP)**
Inventor: **Suzuki, Tadao**
**No. 1, Ishida-Nishinotsubo Fushimi-ku**
**Kyoto-shi Kyoto (JP)**
Inventor: **Motosugi, Kenzo**
**No. 9-2 Uji-Gokashoshinkai**
**Uji-shi Kyoto (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

(56) References cited:
CHEMICAL ABSTRACTS, vol. 92, no. 5, 4th February 1980, page 326, no. 36629a, Columbus, Ohio, U.S.A., M.J. COMER et al.: "Purification and properties of glycerokinase from Bacillus stearothermophilus"
CHEMICAL ABSTRACTS, vol. 88, no. 23, 5th June 1978, page 284, no. 166456v, Columbus, Ohio, U.S.A., B.C. SAHA et al.: "Effect of growth temperature on glucose-6-phosphate dehydrogenase activity in a thermophilic bacterial strain"
CHEMICAL ABSTRACTS, vol. 85, no. 11, 13th September 1976, page 261, no. 74625j, Columbus, Ohio, U.S.A., Y. SUZUKI et al.: "Production of extracellular alpha-glucosidase by a thermophilic Bacillus species"
CHEMICAL ABSTRACTS, vol. 81, no. 17, 28th October 1974, page 182, no. 101122u, Columbus, Ohio, U.S.A., I. IBRAHIM et al.: "Heat stability of lactic and malic dehydrogenase enzymes (LDH and MDH) in some Bacillus species"

2

## Description

The invention relates to a biologically pure culture of strain IFO 14093, and a process for producing an intracellular component herefrom.

Since bacterial cells are smaller in size than yeast or fungal cells, they are difficult to collect from a culture, so they are usually collected by centrifugation rather than by filtration. But the rate of sedimentation of cells in centrifugation is proportional to the square of the cell diameter (see Sekiyu Hakko, *Petroleum Fermentation,* p. 102, 1970, Saiwai Publishing Company), so the recovery of bacterial cells from the fermentation broth is more expensive than recovery of yeast and fungal cells and is very disadvantageous in an industrial operation. According to the estimate by Daniel I. C. Wang, the cost of recovery of bacteria is about 3.8 times the cost of recovery of yeast (see *Chemical Engineering,* Vol. *15,* p. 99, 1968). Therefore, several methods have been proposed for recovery of bacterial cells and among them is flocculation of cells with a flocculant such as ferric chloride, calcium chloride or polymeric flocculant, or modifying the cell protein to an easily collectable form by heating or treatment with a strong acid or base. These techniques are effective when the cell is not the end product, but when the cell per se or the components in the cell are the end product, they are not effective because the flocculant contaminates the product, or the components in the cell are denatured.

Acetate kinase, one example of the endoenzymes, is an enzyme widely used for the synthesis of adenosine triphosphate, a source of biological energy, from acetylphosphoric acid and adenosine diphosphate, or for determination of the acetic acid levels in foods. Acetate kinase is usually obtained as a purified enzyme from *Escherichia coli* (see Journal of Biological Chemistry, *211,* p. 737, 1954, and Methods in Enzymology, *1,* p. 591), but the acetate kinase isolated from *Escherichia coli* is very unstable and not suitable for use on an industrial scale.

Japanese Patent Application (OPI) No. 25088/77 (the term "OPI" as used herein means a published unexamined Japanese patent application) describes a process for producing a highly heat-resistant (i.e., heat-stable) acetate kinase from a thermophilic bacterium *Bacillus stearothermophilus.*

Polynucleotide phosphorylase is used for synthesis of ribo-homopolymers or copolymers of nucleotide, a variety of analog polynucleotides, oligonucleotides having a fixed base arrangement, and for decomposition of RNA. The enzyme also finds much use in the study of the structure and physical properties of nucleic acids. Polynucleotide phosphorylase is conventionally obtained as an extract from *Micrococcus luteus* (see *Procedures in Nucleic Acid Research,* Vol. *2,* p. 896,1971), but the polynucleotide phosphorylase isolated from that microorganism is so labile that it is unsuitable for use even in laboratory-scale research, to say nothing of use on an industrial scale.

"*Seikagaku* (Biochemistry)", Vol. *47,* p. 738 (1975) describes a method of producing a highly heat-stable, heat-resistant polynucleotide phosphorylase from a thermophilic bacterium *Thermus thermophilus,* and *Nucleic Acids Research,* Vol. *3,* p. 219 (1976) describes a method of producing the same enzyme from a thermophilic bacterium *Bacillus stearothermophilus.*

Malate dehydrogenase which produces oxaloacetic acid from malic acid using nicotinamide adenine nucleotide as a coenzyme has recently been much in demand as an enzyme for use in clinical diagnosis. This enzyme is usually obtained as a purified extract from animal tissues, say, bovine heart (see *Methods in Enzymology,* Vol. *13,* p. 99), but malate dehydrogenase isolated from animal tissues is labile and is not easy to handle. Therefore, a supply of a stable malate dehydrogenase was desired, and the *Journal of Biological Chemistry,* Vol. *242,* p. 1548, 1967 and *Biochemical Journal,* Vol. *177,* p. 441, 1979 have described a method of producing a highly heat-stable, heat-resistant malate dehydrogenase from a thermophilic bacterium *Bacillus stearothermophilus.*

Glucokinase and glucose-6-phosphate dehydrogenase are used to measure the glucose or hexokinase level in the body fluid, which is an important factor in clinical analysis. They are also used to determine the glucose or fructose level in foods which is also an important parameter to know in the manufacture of invert sugar. However, known glucokinase and glucose-6-phosphate dehydrogenase are labile, too (see *Methods in Enzymology,* Vol. *42,* pp. 6—39, 1975 ed. by W. A. Wood, and *Advances in Enzymology,* Vol. *48,* pp. 97—191, 1979, ed. by Alton Meister). The only known exception is the glucokinase described in *FEBS Letters,* Vol. *37,* pp. 212—216, 1973; obtained from thermophilic *Bacillus stearothermophilus,* this enzyme is heat-stable and has long storage stability.

Pyruvate kinase is found in all living tissues such as animals, plants, yeast and bacteria that metabolize carbohydrates by glycolysis or fermentation. In the living cells, the enzyme catalyzes the reaction of forming adenosine triphosphate and pyruvic acid from phosphoenolpyruvic acid (PEP) and adenosine diphosphate (ADP), so it is used for determination of PEP and ADP. The conventional crystal of pyruvate kinase is available as an isolate from rabbit muscle or swine heart (see *Methods in Enzymology,* Vol. *1,* p. 435, 1955 and *Journal of Biological Chemistry,* Vol. *234,* p. 2428, 1959), but such pyruvate kinase is so labile that it is not suitable for industrial use. Japanese Patent Application (OPI) No. 9392/78 describes a method of producing a highly heat-stable, heat-resistant pyruvate kinase from a thermophilic *Thermus thermophilus,* and "*Seikagaku* (Biochemistry)", Vol. *44,* p. 649, 1972, describes a method of producing the same enzyme from a thermophilic *Bacillus stearothermophilus.*

To obtain these useful endoenzymes, cultured cells must be first collected by centrifugation or other suitable means before the desired enzyme is extracted from the cells by ultrasonic treatment or physical breaking.

The collection of cultured cells is difficult, as was already mentioned, because of the low specific gravity of bacteria. In addition, bacteria belonging to the genus *Bacillus* have a relatively hard membrane which can be broken so slightly that the efficiency in extraction of a desired enzyme is low. For these reasons, it has been difficult to produce these enzymes on an industrial scale.

Therefore, one object of the invention is to provide a thermophilic bacterium having an easily settleable cell and an easily breakable cell wall.

Another object of the invention is to provide a process for industrial large-scale production of a useful enzyme selected from the group consisting of a heat-resistant acetate kinase, a heat-resistant polynucleotide phosphorylase, heat-resistant malate dehydrogenase, heat-resistant glucokinase, heat-resistant glucose-6-phosphate dehydrogenase and heat-resistant pyruvate kinase.

To achieve these objects, we conducted a screening of naturally occurring microorganisms by Koch's plate culture (for example, see William Burrows, Textbook of Microbiology, 19th ed, P 21, W. B. Saunders Company, USA) in search for a thermophilic bacterium having an easily settleable cell and an easily breakable cell wall. As a result, we found a new strain in manure in Ogura, Uji, Kyoto, Japan. In consideration of its properties, the strain is believed to belong to *Bacillus stearothermophilus,* but the cell is extremely elongated and is several to several tens of times larger than the cell of known *Bacillus stearothermophilus* described in *Bergey's Manual of Determinative Bacteriology.* We have found that the new strain is a thermophilic microorganism that contains various useful enzymes and which has an easily settleable cell and an easily breakable cell wall.

Therefore, this invention provides a biologically pure culture of strain UK 788 that belongs to *Bacillus stearothermophilus,* the cell of which is longer than about 10 microns and which permits easier release of intracellular components than a type culture *Bacillus stearothermophilus* IAM 11001. The invention also provides a process for producing a useful enzyme by culturing cells of UK 788 and recovering the desired enzyme from the culture.

A biologically pure culture of the strain UK 788 of this invention is very advantageous in industrial use because the cultured cells of the strain can easily be collected and are easy to break. According to this invention, a useful enzyme can be obtained efficiently on an industrial scale.

Fig. 1 is a micrograph (150 x) of the cells of the strain of this invention and a type culture *Bacillus stearothermophilus* IAM 11001 after culturing on a nutrient agar slant culture at 60°C for 24 hours; and

Fig. 2 is a graph showing the relation between ultrasonic treatment time and protein leak, i.e., a graph showing a degree of a breaking tendency of the bacterial cell.

The mycological properties of a biologically pure culture of UK 788 of *Bacillus stearothermophilus* are described below. This culture also has been deposited at the IFO Institute on November 10, 1980 and has been assigned the deposition number IFO 14093. For the methods and the compositions of media used in the determination of mycological properties, see the *Manual of Microbiological Methods,* 1957, ed. by Society of American Bacteriologists, McGraw-Hill Book Company, Biseibutsu no Bunrui to Dotei (Classification and Identification of Microorganisms), 1975, ed. by Takeharu Hasegawa, Tokyo Daigaku Shuppan-Kai, and *Baichigaku Kakuron* (Study of Culture Media), 1967, by Toshiichi Sakazaki, Naya Publishing Company. When an agar medium was used in such determinations, it contained 3 wt.% of agar. Morphological observation: culture at 60°C for 24 hours

1. Shape and size of cell: Very long rod, filamentous, 0.8—1.2×10 to more than a hundred microns, sometimes more than several hundreds of microns
2. Pleomorphism: none
3. Motility: none
4. Spores: Cylindrical endospores formed in the center or on the tip of the cell. No bulging sporangia.
5. Gram strain: positive
6. Acid-fast staining: none

State of growth: culture at 60°C for 24 hours
1. Broth-agar plate culture
   Shape: circular
   Periphery: undulate
   Elevation: flat
   Gloss: none
   Surface: rough
   Appearance: semitransparent
2. Broth-agar slant culture
   Growth: good
   Shape: filamentous

3. Broth liquid culture
   Surface growth: slight ring formation
   Turbidity: strong
   Precipitate: small
   Coloring and decoloring: none
4. Broth-gelatin stab culture

A broth containing 30% gelatin was subjected to stab culture at 60°C for a suitable period, followed by cooling to see if the culture was solidified: gelatin was liquefied.

5. Broth-gelatin stab culture
   Shape: beaded
   Surface growth: good
6. Litmus milk: Litmus discoloration occurred at pH 6.0. Milk first solidified, then liquefied.

Physiological properties: Culture at 60°C for 1 to 2 days
   1. Reduction of nitrate: yes
   2. Denitrifying reaction: negative
   3. MR test: positive
   4. VP test: positive
   5. Indole formation: none
   6. Hydrogen sulfite formation: none
   7. Starch hydrolysis: yes
   8. Utilization of citric acid: none
   9. Utilization of nitrate: yes
   10. Utilization of ammonium salt: yes
   11. Pigment formation: none
   12. Urease activity: none
   13. Oxidase activity: yes
   14. Catalase activity: yes
   15. Growth pH: 5.0—8.5
       Optimum pH: 6.0—7.5
   16. Growth temperature: 40—70°C
       Optimum temperature: 50—63°C
   17. Behavior with respect to oxygen: grows well aerobically and grows slightly even under anaerobic conditions
   18. O—F test: negative
   19. Deamination of phenylalanine: negative
   20. Sodium chloride fastness: grows with 5% sodium chloride but cannot grow with 7% sodium chloride
   21. Vitamin requirement: none
   22. Tyrosine decomposition: none

Formation of acid and gas from carbon source: culture at 60°C for 1 to 2 days.

|  | Acid | Gas |
|---|---|---|
| 1. L-arabinose | — | — |
| 2. D-xylose | + | — |
| 3. D-glucose | + | — |
| 4. D-mannose | + | — |
| 5. D-fructose | + | — |
| 6. D-galactose | — | — |
| 7. Maltose | + | — |
| 8. Sucrose | — | — |
| 9. Lactose | — | — |
| 10. Trehalose | + | — |
| 11. D-sorbitol | — | — |
| 12. D-mannitol | — | — |
| 13. Innositol | — | — |
| 14. Glycerin | — | — |
| 15. Starch | — | — |

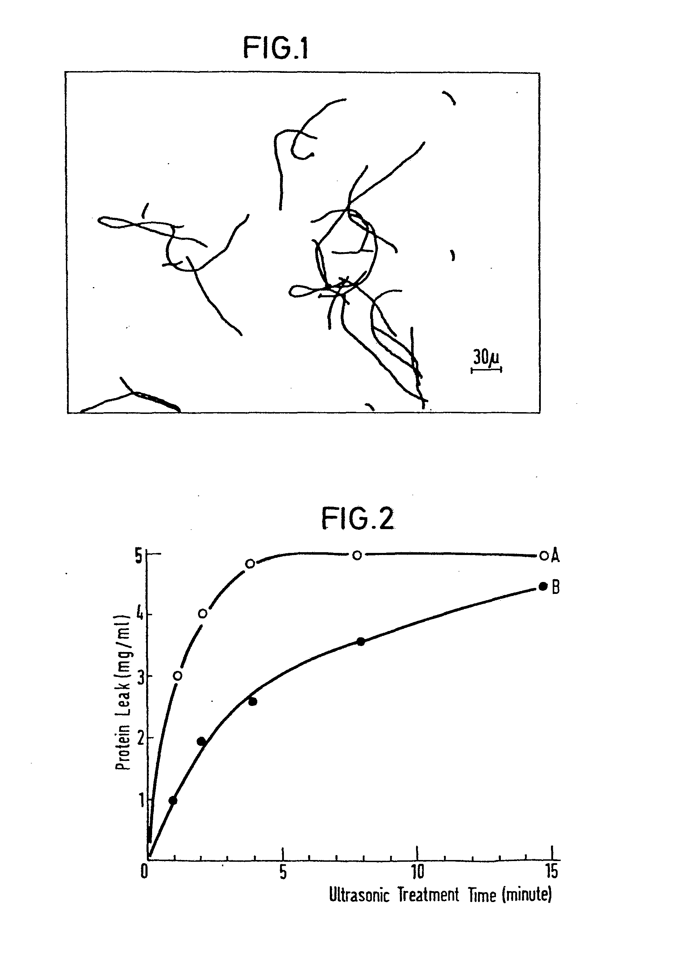

These mycological properties generally agreed with those of *Bacillus stearothermophilus* described in *Bergey's Manual of Determinative Bacteriology,* 8th ed. We therefore compared the strain of this invention with the following type cultures of *Bacillus stearothermophilus,* IAM 11001, 11002, 11003, 11004 (stored at Institute of Applied Microbiology, The University of Tokyo) and IFO 12550 (stored at Institute for Fermentation, Osaka). The new strain UK 788 differed from the type cultures with respect to two or three physiological properties. The biggest difference is in the size of the cell as is clearly seen from Table 1 and Figure 1. Figure 1 is a micrograph (150 x) of the cells of new strain UK 788 and type culture IAM 11001 that were subjected to nutrient slant culture at 60°C for 24 hours. In the micrograph, the cell of new strain UK 788 is seen as a much elongated filament, and the cell of the type culture is seen as a dot or short rod.

The strain of this invention has a cell much more elongated than the cell of the type culture of *Bacillus stearothermophilus,* and *Bacillus stearothermophilus* having a cell comparable to the cell of UK 788 is not described in *Bergey's Manual,* loc. *cit.,* or any other reports. We therefore concluded that the strain used in this invention is completely new and named it, as indicated above, *Bacillus stearothermophilus* UK 788 (hereinafter, referred to as IFO 14093). The strain has been deposited at IFO Institute on November 10, 1980 under a receipt No. IFO 14093.

6

### TABLE 1
Cell size of UK 788 and type culture of *Bacillus stearothermophilus*

| Strain | Cell size* |
|---|---|
| Strain UK 788 | 0.8—1.2×10 to more than hundred microns, sometimes more than several hundreds of microns |
| IAM 11001 | 0.8—1.0×2 to 8 microns |
| IAM 11002 | 0.6—0.8×2 to 5 microns |
| IAM 11003 | 0.6—1.0×2 to 8 microns |
| IAM 11004 | 0.6—1.0×1.5 to 5 microns |
| IFO 12550 | 0.6—1.0×2 to 8 microns |

*The figures were those obtained after nutrient-agar slant culture at 60°C for 24 hours.

Common media for cultivation of bacteria may be used in culturing strain UK 788, and a liquid medium is preferred. The medium can contain a variety of nutrient sources: carbon sources include sugars such as glucose, sucrose, fructose, starch hydrolyzate, molasses and sulfite pulp spent liquor, organic acids such as acetic acid and lactic acid, and also alcohols, fats and oils, aliphatic acids, and glycerin that can be assimilated by strain UK 788; nitrogen sources include inorganic or organic materials such as ammonium sulfate, ammonium chloride, ammonium phosphate, uric acid, ammonia, nitrate, amino acid, peptone, meat extract and yeast extract; inorganic salts such as potassium, sodium, phosphoric acid, zinc, iron, magnesium, manganese, copper, calcium, and cobalt salts. Optionally, traces of metals, corn steep liquor, vitamins, nucleic acid, etc., may also be used. Nutrient sources commonly utilized by bacteria may be used. On a medium containing these nutrient sources, the strain UK 788 of this invention is cultured aerobically for from about 2 to 6 hours, generally at from about 20 to 80°C, preferably at from 40 to 70°C, and more preferably at from 50 to 63°C. Cells containing a heat-resistant acetate kinase can be obtained by either batch culture or continuous culture.

The strain UK 788 of this invention may be used to obtain intracellular components such as sugars, proteins, lipids, nucleic acids and vitamins. Examples of proteins are enzymes and coenzymes; enzymes and coenzymes that are promising as industrial reagents include acetate kinase, ATPase, DNA polymerase, enolase, glucokinase, β-galactosidase, glucose isomerase, lactate dehydrogenase, myokinase, 6-phosphogluconate dehydrogenase, phosphoglycerate kinase, polynucleotide phosphorylase, pyruvate kinase, and superoxide dismutase.

A useful enzyme may be produced from strain UK 788 of this invention by either batch cultivation or continuous cultivation. Batch culture is preferably continued to the last stage of the logarithmic growth phase. Continuous culture is preferably conducted by the substance environmental-type continuous cultivation method (chemostat; Herbert D., Ellsworth R. and Telling R. C., *Journal of General Microbiology*, Vol. *14*, No. 8 pp. 601—622, 1956) and cells having a high content of the desired useful enzyme can be obtained by adjusting the dilution ratio (the rates of supply of liquid medium to fermentation tank and withdrawal therefrom divided by the volume of liquid medium in the fermentation tank) close to the maximum specific growth rate of strain UK 788.

A desired useful enzyme can be isolated and purified from the culture by the following procedures: lhe cells are first collected from the culture by centrifugation or filtration, i.e., the treatment of collecting the cells is carried out by centrifuging the culture with Sharples or De Laval type centrifuge or by filtering the culture by means of a constant-pressure filtration or a rotating drum filtration (cf. *Biochemical Engineering*, Second Edition, pages 349 to 355, 1973, ed. by Shuichi Aiba, Arthur E. Humphrey, Nancy F. Millis, University of Tokyo Press), and the collected cells are subjected to a conventional enzyme isolation and purification technique, i.e., the cells are crushed and centrifuged to provide a supernatant which is either fractionated with an organic solvent or a variety of salts such as sodium chloride, magnesium sulfate, ammonium sulfate, sodium sulfate, potassium phosphate, sodium citrate, and so forth, or purified by adsorption on a carrier. An example of such a conventional technique is described in *Journal of Biochemistry*, Vol. *84*, No. 1, pp. 193—203, 1978.

One example of a method for producing a heat-resistant polynucleotide phosphorylase is described in *Nucleic Acids Research*, Vol. *3*, p. 219, 1976; an example of a technique for producing a heat-resistant malate dehydrogenase is described in *Biochemical Journal*, Vol. *177*, p. 441, 1979.

One method of producing a heat-resistant glucokinase is described below: cultured cells are suspended in a 50 millimole/l (hereinafter, referred to as "mM") phosphate buffer (pH=7.5), in an amount by weight twice as much as the weight of the cells, and the cells are thoroughly crushed with a French press; the cell fragments are removed by centrifugation to provide a crude extract containing glucokinase; 3.2 wt.% aqueous protamine sulfate is added to the crude extract so that the concentration of the protamine sulfate is 0.5 wt.% and the mixture is thoroughly stirred; the resulting precipitate is removed by centrifugation to provide a protamine supernatant; solid ammonium sulfate is gradually added to the supernatant until it is 50% saturated (4°C), and the resulting precipitate is collected by centrifugation and again dissolved in 50 mM phosphate buffer (pH=7.5) and desalted by dialysis with 50 mM phosphate buffer (pH=7.5) in 20-times amount per total amount of the 50 mM phosphate buffer solution of the precipitate; then, the crude extract is passed through a DEAE-Cellulose column equilibrated with 50 mM phosphate buffer (pH=7.5), and upon elution with a solution composed of potassium chloride and a phosphate buffer of the same composition as used above, the desired glucokinase is obtained at a KCl concentration of about 0.13 M or so. Active fractions are combined, concentrated, desalted and passed through a hydroxyapatite column equilibrated with 10 mM phosphate buffer (pH=7.5); upon elution with a phosphate buffer having a linear gradient of from 10 mM to 300 mM, the desired glucokinase is obtained at a concentration of about 120 mM. The active fraction is concentrated and fractionated by column chromatography on Sephadex G-100 using, 50 mM tris-HCl buffer (pH=8.0) as an eluting agent.

One example of the method of producing a heat-resistant glucose-6-phosphate dehydrogenase is as follows: cultured cells are suspended in 0,1 M phosphate buffer (pH=7.5) in an amount by weight twice as much as the weight of the cells, and the cells are thoroughly crushed with a French press; the cell fragments are removed by centrifugation to provide a crude extract containing glucose-6-phosphate dehydrogenase; 600 ml of the crude extract is dissolved in 300 ml of 1 wt% aqueous protamine sulfate and the mixture is thoroughly stirred; the resulting precipitate is removed by centrifugation to provide a protamine supernatant; solid ammonium sulfate is gradually added to the supernatant until it is 50% saturated (4°C), and the resulting precipitate is collected by centrifugation and again dissolved in 0.1 M phosphate buffer (pH=7.5) and desalted by dialysis with 0.1 M phosphate buffer (pH=7.5) in 20-times amount per total amounts of the 0.1 M phosphate buffer solution of the precipitate; then, the crude extract is passed through a DEAE-Cellulose column equilibrated with 20 mM phosphate buffer (pH=7.5) containing 2 mM mercaptoethanol and 2 mM sodium ethylenediaminetetraacetate, and upon elution with a solution composed of potassium chloride and a buffer of the same composition as used above, the desired glucose-6-phosphate dehydrogenase is obtained at a KCl concentration of about to 0.15 M. Active fractions are combined, concentrated, desalted and passed through a hydroxyapatite column equilibrated with 5 mM phosphate buffer (pH=7.5); upon elution with a phosphate buffer having a linear gradient of from 5 mM to 250 mM, the desired glucose-6-phosphate dehydrogenase is obtained at a concentration near 75 mM. The active fraction is concentrated, desalted and fractionated by column chromatography on ultrogel ACA 34 using an eluting agent composed of 50 mM tris-HCl buffer containing 0.1 M potassium chloride. The active fraction is then passed through a DEAE-Sephadex A-50 column equilibrated with 30 mM phosphate buffer (pH=7.7) containing 2 mM mercaptoethanol and 2 mM sodium ethylenediaminetetraacetate, and eluted with a buffer solution (of the same composition as above) plus potassium chloride having an KCl linear gradient of 0.1 M.

One example of a method of producing a heat-resistant pyruvate kinase is described in "Seikagaku (Biochemistry)", Vol. 44 p. 649, 1972.

These useful enzyme obtained can be purified by the techniques described above, and the physicochemical properties of the resulting crystals and the mechanism of their actions can be compared with those of the crystals obtained from other strains of Bacillus stearothermophilus; the heat-resistant acetate kinase obtained according to the present invention has the same properties as the ones described in Journal of Biochemistry, Vol. 84, No. 1, pp. 193—203, 1978 and Japanese Patent Application (OPI) No. 25088/77, the heat-resistant polynucleotide phosphorylase obtained according to the present invention has the same properties as the one described in Nucleic Acids Research, Vol. 3, p. 219, 1976, the heat-resistant maleate dehydrogenase obtained according to the present invention has the same properties as the one described in Biochemical Journal, Vol. 177, p. 441, 1979; the heat-resistant glucokinase obtained according to the present invention has the same properties as the one described in FEBS Letters, Vol. 37, p. 212, 1973; and the heat-resistant pyruvate kinase obtained according to the present invention has the same properties as the ones described in Seikagaku (Biochemistry), Vol. 44, p. 649, 1972 and Japanese Patent Application (OPI) No. 9392/78.

The physicochemical properties of the crystal of the heat-resistant glucose-6-phosphate dehydrogenase and the mechanism of its action are specifically described below.

The heat-resistant glucose-6-phosphate dehydrogenase according to this invention is a glucose-6-phosphate wherein the maximum residual activity of the enzyme, when treated in a buffer at about 50°C for about 15 minutes, can be maintained at about 80% or more, preferably about 90% or more, and more preferably about 100%, based on the original activity. The concentration and pH of the buffer are not limited to particular values, and generally, the concentration is between 5 mM and 500 mM, and the pH is between 7 and 10.5. For the purposes of this invention, 100 mM tris-HCl buffer (pH=ca. 9.0) containing 100 mM potassium chloride is preferred.

## 1. Activity

The glucose-6-phosphate dehydrogenase catalyzes a reaction represented by the following scheme:

Hexose-6-phosphate+coenzyme (oxidation type)

⇅

Hexose · aldonic acid-6-phosphate+coenzyme (reduction type)

In these schemes, the hexose-6-phosphate is a generic term for glucose-6-phosphate, mannose-6-phosphate and galactose-6-phosphate, and the hexose · aldonic acid-6-phosphate is a generic term for gluconic acid-6-phosphate, mannoic acid-6-phosphate and galactonic acid-6-phosphate. The coenzyme is either NADP or NAD.

## 2. Substrate specificity

The glucose-6-phosphate has a Michaelis constant (Km value) of about 0.16 mM. The reaction rates for a given concentration of substrates mannose-6-phosphate and galactose-6-phosphate are about 40% and 20%, respectively, of the rate for glucose-6-phosphate. The Km values for coenzymes NADP and NAD are about 0.016 mM and 1.64 mM, respectively.

## 3. Optimum pH

About 9.0 (at 30°C)

## 4. Stable pH range

Little inactivation occurs at a pH of 7.0 to 10.5 even if the enzyme is treated at 4°C for 24 hours.

## 5. Temperature range suitable for exhibiting enzyme activity

The activity increases with a temperature increase from 25 to 75°C at a pH of 8.9. In most recent cases, the enzyme is used to catalyze the reaction at 30°C.

## 6. Heat resistance

The enzyme is stable against heating at 57°C for 15 minutes.

## 7. Molecular weight

Gel chromatography on Sephacryl S-200 showed that the enzyme had a molecular weight of about 230,000. The glucose-6-phosphate dehydrogenase from yeast or *Leuconostoc mesenteroides* had a molecular weight of about 100,000 in the same analysis.

## 8. Determination of enzyme activity

To a mixture of 2 mM glucose-6-phosphate, 0.5 mM NADP, and 5 mM magnesium chloride in 50 mM tris-HCl buffer (pH=8.9), a suitable amount of glucose-6-phosphate dehydrogenase was added, and the activity of the enzyme was determined on the basis of the increase in the absorbance at 340 nm of NADP reduction type (H) for a given time. The activity required to increase the absorbance of 1 micromol of NADPH at 340 nm per minute was defined to be one unit. A purified glucose-6-phosphate dehydrogenase had an activity of about 100 units per mg (of purified enzyme) at 30°C. Since the glucose-6-phosphate dehydrogenase of this invention is highly heat-resistant, it is capable of catalyzing the desired reaction at a temperature between about 50 and 60°C, at which the previously used glucose-6-phosphate dehydrogenase is inactivated. The activity in that temperature range is even higher.

## 9. Purity

In a disc electrophoresis in 7.5% acrylamide at pH 9.4, the purified product of the glucose-6-phosphate dehydrogenase moved to the positive electrode and gave a single band. In SDS electrophoresis, the enzyme also moved to the positive electrode and gave a single band.

## 10. Compositional analysis

The proportions of the amino acids in the glucose-6-phosphate dehydrogenase are set forth below:

Aspartic acid 11.04%, threonine 5.24%, serine 4.56%, glutamic acid 11.86%, proline 3.66%, glycine 6.67%, alanine 7.92%, cystine (cysteine) 0.62%, valine 6.09%, methionine 1.97%, isoleucine 5.59%, leucine 8.04%, tyrosine 3.72%, phenylalanine 4.88%, lysine 5.28%, histidine 3.40%, arginine 6.56%, tryptophan 2.72%.

## 11. Crystalline structure

The glucose-6-phosphate dehydrogenase has yet to be crystallized, so its crystalline structure has not been determined.

The cells of UK 788 cultured and harvested by the method of this invention are very easy to collect, and the centrifugation time, that was 10 minutes at 8000 G with the known strains of *Bacillus stearothermophilus,* is reduced to about a fifth thereof. This offers a great merit because it permits so far impractical cell collection by filtration through a filter medium. The cells of the known *Bacillus stearothermophilus* required 15 minutes of ultrasonic treatment (Frequency: 10 KHz, Output: 200 W) to break, but the collected cells of UK 788 can be equally broken by an ultrasonic treatment that lasts for only about 3 minutes under the same conditions. This also presents a significant advantage in the industrial production of a useful enzyme.

This invention is now described in greater detail by reference to the following examples which are given here for illustrative purposes only and are by no means intended to limit the scope of the invention.

Example 1 and Comparative Example 1

A 500-ml conical flask was charged with 100 ml of a medium prepared by dissolving in one liter of tap water a mixture of 2 g of glucose, 2 g of $(NH_4)_2SO_4$, 1 g of yeast extract (Difco), 1 g of $KH_2PO_4$ 1 g of $Na_2HPO_4 \cdot 12H_2O$ and 0.1 g of $MgSO_4 \cdot 7H_2O$, closed up with a cotton stopper and sterilized with pressurized steam (121°C, latm.) for 10 minutes. The medium was cooled to 50°C and inoculated with *Bacillus stearothermophilus* UK 788 grown on a nutrient agar slant medium of the same formulation as indicated above, and subjected to rotary shake cultivation in a rotary shaker (RGR No. 2 type shaker of Takasaki Seisakusho, 180 rpm) at 55°C. After conducting the rotary shake cultivation for 5 hours, when the growth of cells was observed, the turbidity of the medium was had an absorbance of 1.0 at 660 nm (measured by 101-type spectrophotometer produced by Hitachi, Ltd.) and the growth of cells entered the last stage of the logarithmic growth phase, the cultivation was stopped and the cells were collected by centrifugation with a centrifuge (RS 71 of Tomy Seisakusho) for 2 minutes at 8000 G. The yield of cells collected was determined. One gram of the wet cells was suspended in 20 ml of 0.1 M phosphate buffer (pH 7.0) and the protein that leaked from the cells treated with an ultrasonic breaker (Model 200 M of Kubota Medical Appliance Supply Corp.) was measured.

In Comparative Example 1, the procedure of Example 1 was repeated, except that *Bacillus stearothermophilus* IAM 11001 was used.

It was confirmed that the cells of UK 788 could be recovered from the culture medium by centrifugation more easily than the cells of IAM 11001; the percent recovery of UK 788 was 98% and that of IAM 11001 was 42%.

Figure 2 represents the relation between ultrasonic treatment time and protein leak. In the figures, curve A represents Example 1 and curve B represents Comparative Example 1. As is clear from Figure 2, the cells used in Example 1 could be broken and intracellular protein leaked by an ultrasonic treatment required only about a fifth of the period required to break the cells used in Comparative Example 1. The protein leak was determined by the biuret method (see Gornall A. G., *Journal of Biological Chemistry,* Vol. *177,* p. 751, 1949).

Example 2

Culture was performed in ten 500-ml conical flasks using a medium of the same composition as used in Example 1. The liquid cultures were combined, transferred to a 30-liter jar fermentor (MSJ-U Model of Marubishi Rika Sochi, using a straight impeller turbine) that contained 20 liters of a medium of the same composition and which had been sterilized with pressurized steam (121°C, latm., 15 minutes), and subjected to fermentation at 60°C and 400 rpm with air supplied at a rate of 20 liters/min. Cell growth was soon observed and a drop in pH occurred. The fermentation was continued for another 3 hours while 4N NaOH was used to maintain the pH at 6.5. When the cell growth entered the last stage of the logarithmic growth phase, the culture was stopped and the medium was continuously centrifuged with a centrifuge (LAPX 202 of Alpha Laval) at 8000 G for 2 minutes, to provide 120 g of wet cells in a yield of 97%. In the same manner as in Example 1, the relation between the ultrasonic treatment time and intracellular protein leak was examined, and the relative ratio of protein leaked upon a 3-minute treatment was 90%, indicating that the strain UK 788 had easily settleable cells and an easily breakable cell wall even after bench-scale fermentation.

Example 3

Twenty grams of the wet cells produced in Example 2 were suspended in 200 ml of 0.1 M phosphate buffer (pH 7.5) and treated with an ultrasonic breaker for 3 minutes. The resulting solution containing broken cells were centrifuged at 8000 G for 2 minutes to obtain 202 ml of a supernatant free from residual cells. Under cooling at 4°C, 1.2 g of protamine sulfate was added to the supernatant, and the nucleic acid precipitate was removed by centrifugation. When ammonium sulfate was added to the supernatant until it was 75% saturated, protein was salted out and recovered by centrifugation. The protein precipitate was dissolved in 20 ml of 0.1 M phosphate buffer (pH 7.5), and the solution was desalted by dialysis through a Cellophane membrane against 2 liters of 0.1 M phosphate buffer (pH 7.5) for 4 hours. The desalted solution was fractionally purified to obtain acetylkinase and ATPase by chromatography on a DEAE-Cellulose column, chromatography on a hydroxyapatite column, and chromatography on an ultrogel column (see *Journal of Biochemistry,* Vol. *84,* No. 1, pp. 193—203, 1978). As a result, electrophoretically pure acetylkinase (0.82 mg) and ATPase (0.32 mg) were obtained, confirming that the cells of UK 788 were a good source for the supply of useful endoenzymes.

Example 4

The liquid cells broken by ultrasonic treatment in Example 1 contained 97.2 U of heat-resistant acetate kinase per gram of wet cell (1.2 U/mg) of protein and this figure was almost equal to the level (93.5 U/g wet cell) of heat-resistant acetate kinase in the cells obtained in Comparative Example 1.

The content of the heat-resistant acetate kinase produced was measured by the reverse reaction system described in *Journal of Biological Chemistry,* Vol. *249,* p. 2567, 1974: changes in ATP are converted to changes in nicotinamide adenine dinucleotide reduced form (hereunder referred to as NADH) and these changes are traced by absorbance at 340 nm. The enzymatic activity required to reduce the absorbance of 1 micromol of NADH at 340 nm per minute is defined to be one unit (hereunder referred to as U).

Example 5

The level of heat-resistant acetate kinase in the wet cells produced in Example 2 was determined as in Example 3, and it was found to be 105.3 U per gram of wet cells (1.3 U per mg of protein).

Example 6

Batch culture was performed in a 30-liter jar fermentor in the same manner as in Example 2. When the cell growth entered the last stage of the logarithmic growth phase and the residual glucose level in the liquid culture was less than 0.01 wt%, a chemostatic fermentation was performed with the dilution ratio being held close to the maximum specific growth rate of the microorganism by supplying a fresh medium (of the same composition as used in Example 1) to the fermentor and withdrawing the fermentation liquor from the fermentor with a metering pump at a rate of 24 liters per hour. The other culture conditions were as follows: temperature, 60°C; pH, 6.8—7.0 (controlled automatically with 4N NaOH), air supply rate, 20 liters per min.; and stirring speed, 600 rpm. During the fermentation, foaming occurred, so a defoaming agent (KM-70 of Shinetsu Chemical Industry Co., Ltd.) was added. Throughout the continuous fermentation that lasted for about 4 hours, the cell concentration was maintained at the level achieved at the start of the fermentation (5.8 g of wet cells per liter or 0.75 g of dry cell per liter), and 550 g of wet cells were centrifuged from 96 liters of the fermentation liquor. The cells so obtained contained 130 U of heat-resistant acetate kinase per gram of wet cells (1.6 U per mg of protein). The cells harvested by continuous fermentation contained more heat-resistant acetate kinase per unit cell than those produced solely by batch processing.

Example 7

The liquid cells broken by ultrasonic treatment in Example 1 contained 1500 U of heat-resistant polynucleotide phosphorylase per mg of wet cells (1.5 U per mg of protein). This figure was substantially equal to the level of heat-resistant polynucleotide phosphorylase in the cells obtained in Comparative Example 1. The level of heat-resistant polynucleotide phosphorylase was determined by chlorimetric analysis of phosphoric acid freed from adenosine diphosphate when oligoadenosine was used as a primer *(Nucleic Acids Research,* Vol. *3,* p. 219, 1976). The enzymatic activity required to free 1 micromol of phosphoric acid per hour was defined to be one unit (hereunder referred to as U).

Example 8

The level of heat-resistant polynucleotide phosphorylase in the cell produced in the same manner as in Example 6 was determined. As a result, the cells so obtained contained 1,900 U of heat-resistant polynucleotide phosphorylase per gram of wet cells (1.8 U per mg of protein). The cells harvested by continuous fermentation contained more heat-resistant polynucleotide phosphorylase per unit cell than those produced solely by batch processing.

Example 9

To the solution containing broken cells that was obtained by ultrasonic treatment in Example 1, 0.6 wt% of protamine sulfate was added to precipitate nucleic acids which were then removed by centrifugation at 8000 G for 10 minutes. The resulting supernatant or crude enzyme extract contained 0.05 U of heat-resistant malate dehydrogenase per mg of protein, which figure was substantially equal to the activity of heat-resistant maleate dehydrogenase in the cells produced in Comparative Example 1 (0.06 U/mg of protein).

The level of heat-resistant maleate dehydrogenase was determined by the method described in *Journal of Biological Chemistry,* Vol. *242,* p. 1548, 1967: changes in oxaloacetic acid are converted to changes in nicotinamide adenine dinucleotide reduction type (hereunder referred to as NADH), and these changes are traced by measuring the absorbance at 340 nm, and the enzymatic activity required to reduce the absorbance of 1 micromol of NADE at 340 nm per minute is defined to be one unit (hereunder referred to as U).

Example 10

The cells of UK 788 were cultured and collected in the same manner as in Example 6. The level of heat-resistant maleate hydrogenase contained in 1 g of the wet cells was determined as in Example 9, and it was found to be 0.09 U per mg of protein. The cells harvested by continuous fermentation contained more heat-resistant malate dehydrogenase per unit cell than those produced by batch process.

Example 11

The solution containing broken cells that was obtained by an ultrasonic treatment in Example 1 contained 9.5 U of a heat-resistant glucokinase per gram of wet cells (0.12 U per mg of protein), which figure was substantially equal to the level of heat-resistant glucokinase in the cells produced in Comparative Example 1.

Example 12

The wet cells obtained in Example 2 contained 10.3 U of heat resistant glucokinase per gram of wet cells (0.13 U per mg of protein).

Example 13

The wet cells obtained by repeating the procedure of Example 5 contained 12.8 U of heat-resistant glucokinase per gram of wet cells (0.16 U per mg of protein). The cells harvested by continuous fermentation contained more heat-resistant glucokinase per unit cell than those produced solely by batch processing.

Example 14

The solution containing broken cells that was obtained by ultrasonic treatment in Example 1 contained 3.9 U of a heat-resistant glucose-6-phosphate dehydrogenase per gram of wet cells (0.14 U per mg of protein), which figure was substantially equal to the level of heat-resistant glucose-6-phosphate dehydrogenase in the cells produced in Comparative Example 1.

Example 15

The wet cells obtained in Example 2 contained 4.2 U of a heat-resistant glucose-6-phosphate dehydrogenase per gram of wet cells (0.17 U per mg of protein).

Example 16

The wet cells obtained by repeating the procedure of Example 5 contained 4.8 U of a heat-resistant glucose-6-phosphate dehydrogenase per gram of wet cells (0.2 U per mg of protein). The cells harvested by continuous fermentation contained more heat-resistant glucose-6-phosphate dehydrogenase per unit cell than those produced by batch processing.

Example 17

The solution containing broken cells that was obtained by ultrasonic treatment in Example 1 contained 34.7 U of a heat-resistant pyruvate kinase per gram of wet cells (0.44 U per mg of protein), which figure was substantially equal to the level of heat-resistant pyruvate kinase in the cells produced in Comparative Example 1. The level of heat-resistant pyruvate kinase was determined by measuring the decrease in the absorbance at 340 nm of nicotinamide adenine dinucleotide reduced form (NADH) that was used to reduce pyruvic acid freed from PEP upon transfer of a phosphate group to ADP. The enzymatic activity required to reduce the absorbance of 1 micromol of NADH at 340 nm per minute was defined to be one unit (hereunder referred to as U).

Example 18

Wet cells of UK 788 were produced as in Example 2, and the level of heat-resistant pyruvate kinase in the cells was determined as in Example 17, and it was found to be 37.6 U per gram of wet cell (0.48 U per mg of protein).

Example 19

Wet cells of UK 788 were produced as in Example 5, and the level of heat-resistant pyruvate kinase in the cells was determined as in Example 17, and it was found to be 46.4 U per gram of wet cell (0.59 U per mg of protein). The cells harvested by continuous fermentation contained more heat-resistant pyruvate kinase per unit cell than those produced solely by batch processing.

While the invention has been described in detail and with reference to specific embodiments thereof, it will be apparent to one skilled in the art that various changes and modifications can be made therein without departing from the spirit and scope thereof.

**Claims**

1. A biologically pure culture of *Bacillus stearothermophilus* IFO No. 14093 the cell of which is longer than about 10 microns and which permits easy release of an intracellular component.

2. A process of extracting an intracellular component from a biologically pure culture of *Bacillus stearothermophilus* IFO No. 14093, comprising culturing cells of this strain and recovering the desired intracellular component from the culture.

3. A process according to claim 2 wherein the intracellular component is an enzyme selected from the group consisting of a heat-resistant acetate kinase, a heat-resistant polynucleotide phosphorylase, heat-resistant malate dehydrogenase, heat-resistant glucokinase, heat-resistant glucose-6-phosphate dehydrogenase and heat-resistant pyruvate kinase.

4. A process according to claim 2 wherein the culture is performed by a batch process until the cell growth has entered the last stage of the logarithmic growth phase.

5. A process according to claim 4 wherein continuous culture is performed with a dilution ratio held close to the maximum specific growth rate of the strain of claim 1.

6. A process as in claim 2 or 4 wherein the culturing is conducted in a liquid medium.

7. A process as in claim 5 wherein the culturing is conducted in a liquid medium.

8. A process as in claim 2, 4 or 6 wherein the culturing is conducted aerobically for from about 2 to 6 hours at a temperature of from about 20 to 80°C.

9. A process as in claim 8 wherein the temperature is from 40 to 70°C.

10. A process as in claim 8 wherein the temperature is from 50 to 63°C.

11. A process as in claim 2 wherein the intracellular component is a protein and an enzyme.

**Patentansprüche**

1. Biologisch reine Kultur von *Bacillus stearothermophilus* IFO Nr. 14093, wobei die Zellen nicht länger als etwa 10 $\mu$m sind und die eine einfache Freigabe einer intrazellularen Komponente ermöglicht.

2. Verfahren zum Extrahieren einer intrazellularen Komponente von einer biologisch reinen Kultur von *Bacillus stearothermophilus* IFO Nr. 14093, dadurch gekennzeichnet, dass man Zellen dieses Stammes kultiviert und die gewünschte intrazellulare Komponente aus der Kultur gewinnt.

3. Verfahren gemäss Anspruch 2, dadurch gekennzeichnet, dass die intrazellulare Komponente ein Enzym ist, ausgewählt aus der Gruppe bestehend aus einer wärmebeständigen Acetatkinase, einer wärmebeständigen Polynucleotidphosphorylase, einer wärmebeständigen Malatdehydrogenase, einer wärmebeständigen Glucokinase, einer wärmebeständigen Glucose-6-phosphat-dehydrogenase und einer wärmebeständigen Pyruvatkinase.

4. Verfahren gemäss Anspruch 2, dadurch gekennzeichnet, dass man die Kultivierung absatzweise durchführt bis das Zellwachstum in das letzte Stadium der logarithmischen Wachstumsphase eingetreten ist.

5. Verfahren gemäss Anspruch 4, dadurch gekennzeichnet, dass man die kontinuierliche Kultivierung bei einem Verdünnungsverhältnis, das in der Nähe der maximalen spezifischen Wachstumsrate des Stammes von Anspruch 1 gehalten wird, durchführt.

6. Verfahren gemäss Ansprüchen 2 oder 4, dadurch gekennzeichnet, dass man die Kultivierung in einem flüssigen Medium durchführt.

7. Verfahren gemäss Anspruch 5, dadurch gekennzeichnet, dass man die Kultivierung in einem flüssigen Medium durchführt.

8. Verfahren gemäss Ansprüchen 2, 4 oder 6, dadurch gekennzeichnet, dass man die Kultivierung aerob während etwa 2 bis 6 Stunden bei einer Temperatur von etwa 20 bis 80°C durchführt.

9. Verfahren gemäss Anspruch 8, dadurch gekennzeichnet, dass die Temperatur 40 bis 70°C beträgt.

10. Verfahren gemäss Anspruch 8, dadurch gekennzeichnet, dass die Temperatur 50 bis 63°C beträgt.

11. Verfahren gemäss Anspruch 2, dadurch gekennzeichnet, dass die intrazellulare Komponente ein Protein und ein Enzym ist.

**Revendications**

1. Une culture biologiquement pure de *Bacillus stearothermophilus* IFO n° 14093, dont la cellule a une longueur supérieure à 10 microns environ et qui permet une libération facile d'un composant intracellulaire.

2. Un procédé pour extraire un composant intracellulaire d'une culture biologiquement pure de *Bacillus stearothermophilus* IFO n° 14093, qui consiste à cultiver des cellules de cette souche et à recueillir le composant intracellulaire recherché à partir de la culture.

3. Un procédé selon la revendication 2 dans lequel le composant intracellulaire est une enzyme choisie dans le groupe consistant en une acétate kinase résistant à la chaleur, une polynucléotide phosphorylase résistant à la chaleur, une malate déshydrogénase résistant à la chaleur, une glucokinase résistant à la chaleur, une glucose-6-phosphate déshydrogénase résistant à la chaleur et une pyruvate kinase résistant à la chaleur.

4. Un procédé selon la revendication 2 dans lequel la culture est effectuée en discontinu jusqu'à ce que les cellules passent au dernier stade de la phase de croissance logarithmique.

5. Un procédé selon la revendication 4 dans lequel la culture est effectuée en continu avec un rapport de dilution maintenu près de la vitesse de croissance spécifique maximale de la souche de la revendication 1.

6. Un procédé selon la revendication 2 ou 4 dans lequel la culture est réalisée dans un milieu liquide.

7. Un procédé selon la revendication 5 dans lequel la culture est effectuée dans un milieu liquide.

8. Un procédé selon la revendication 2, 4 ou 6, dans lequel la culture est effectuée dans des conditions aérobies pendant une durée d'environ 2 à 6 heures à une température d'environ 20 à 80°C.

9. Un procédé selon la revendication 8 dans lequel la température est de 40 à 70°C.

10. Un procédé selon la revendication 8 dans lequel la température est de 50 à 63°C.

11. Un procédé selon la revendication 2, dans lequel le composant intracellulaire est une protéine ou en enzyme.

## FIG.1

30μ

## FIG.2

Protein Leak (mg/ml)

A

B

Ultrasonic Treatment Time (minute)